# EUROPEAN PATENT SPECIFICATION

(11) **EP 3 071 001 B1**
(45) Date of publication and mention of the grant of the patent: **31.03.2021**
(21) Application number: 14862865.4
(22) Date of filing: 10.11.2014
(51) Int. Cl.: H05H 6/00, A61N 5/10, H05H 3/06

(54) **TARGET FOR NEUTRON GENERATION**
TARGET ZUR NEUTRONENERZEUGUNG
CIBLE POUR GÉNÉRATION DE NEUTRONS

(30) Priority: 12.11.2013 JP 2013234078
(43) Date of publication of application: 21.09.2016
(73) Proprietor: Cancer Intelligence Care Systems, Inc., Tokyo 135-0063 (JP)
(72) Inventor: SHIODA, Shigeo, Hiratsuka-shi Kanagawa 254-0076 (JP); NAKAMURA, Masaru, Tokyo 135-0063 (JP)
(74) Representative: Weickmann & Weickmann PartmbB
(86) International application number: PCT/JP2014/079709
(87) International publication number: WO 2015/072421

(56) References cited:
- JP-A- S60 246 600
- JP-A- 2014 081 211
- 'Heisei 24 Nendo Kadai Kaiketsugata Iryo Kiki To Kaihatsu Jigyo 'Saihatsu Gan Chiryo no Tameno Shin Sozai Target Gijutsu o Mochiita Kasokukigata Chuseishi Hosoku Ryoho System no Kaihatsu' KENKYU SEIKA HOKOKUSHO February 2013, pages 1 - 23, XP008183120

## Description

### Technical Field

The present invention relates to a target for neutron generation. More specifically, the present invention relates to a target for neutron generation, which includes a palladium layer and a lithium layer on a substrate and generates neutrons by proton irradiation of the lithium layer, and also has excellent durability and is capable of maintaining the neutron generation capability for a long period of time.

### Background Art

Recently, as novel methods for cancer treatment, practical applications of treatment methods by use of neutrons are expected. Atypical example of the method is boron-neutron capture therapy (BNCT). This treatment method is advantageous in that a boron drug having reactivity to neutrons (thermal neutrons) is previously incorporated into cancer cells, and the affected part is irradiated with neutrons, whereby only cancer cells can be selectively killed while the damage to healthy cells is suppressed.

The neutron capture therapy utilizes neutrons as described above, and thus the treatment equipment is generally placed in a nuclear reactor facility. However, nuclear reactor facilities are different from medical facilities and thus inconvenient in terms of handling the patient, etc., which has been an obstacle to the popularization.

Thus, studies have been made on neutron generators that allow generation/use of neutrons without relying on nuclear reactors. Such a neutron generator mainly includes an accelerator and a target that generates neutrons in response to irradiation with charged particles, such as protons, generated from the accelerator.

A target for neutron generation in a neutron generator includes a lithium or like metal layer as a neutron generation source. The lithium layer is bombarded by high-energy charged particles, whereby a nuclear spallation reaction occurs to emit neutrons. As targets for neutron generation, plate-shaped ones and conical ones as described in Patent Document 1 are known. The target for neutron generation of Patent Document 1 is formed in a conical shape having an angle calculated to effectively emit neutrons generated by irradiation with charged particles.

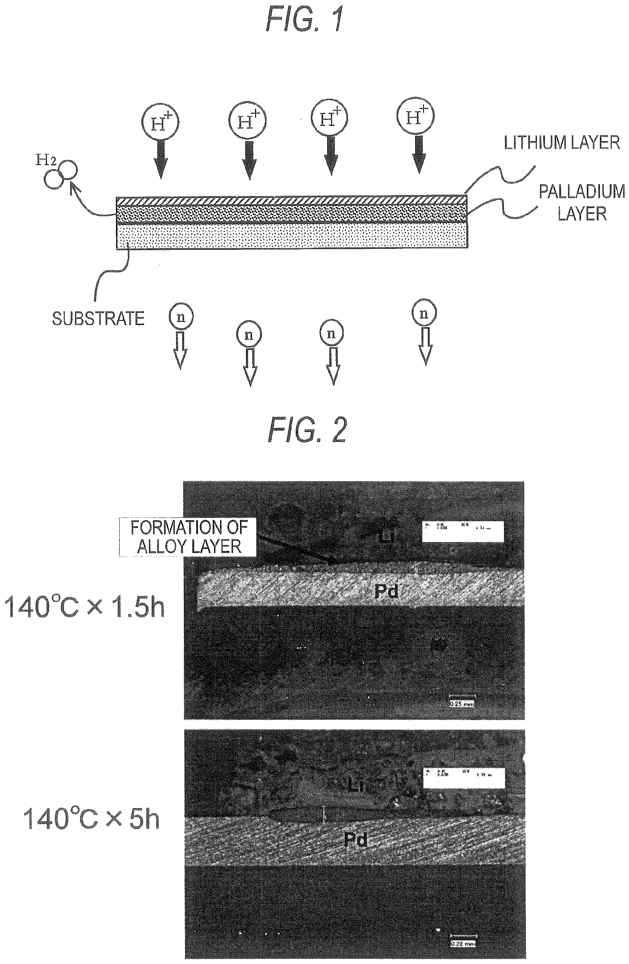

Fig. 1 shows the sectional structure of a target for neutron generation. The target for neutron generation is configured such that a substrate is coated with a lithium layer, which is a proton irradiation surface. The substrate is often made of copper. On the back side of the substrate, considering heat generation due to the nuclear spallation reaction and the melting point of lithium (180°C), a water-cooling channel (not illustrated) for cooling during operation is formed.

Additionally, a palladium layer is formed between the lithium layer and the substrate. This palladium layer is formed to inhibit protons that have been applied and passed through the lithium layer from reaching copper (substrate). This is because when there is no palladium layer, hydrogen that has passed through the lithium layer reaches the copper substrate and, due to the low hydrogen solubility of copper, turns into hydrogen gas, which causes blistering in the surface part of the copper substrate, resulting in separation in the copper surface part. Thus, a palladium layer capable of storing/releasing hydrogen is provided to store hydrogen from the lithium layer, release the absorbed hydrogen from its end, and inhibit hydrogen from reaching the copper substrate.

A target comprising a copper substrate coated with a palladium layer on which a lithium layer is deposited is also known from Non Patent Literature 1. Non Patent Literature 1 furthermore addresses the problem of the formation of an eutectic alloy between the lithium and the palladium layers.

### Related Art Document

### Patent Documents

Patent Document 1: US 2010/0067640 A
Non Patent Literature 1: "Using new material targeting technology for the treatment of recurrent cancer; Development of accelerator-type neutron capture therapy system", research result report (summary version) February 2013, Trustee Ministry of Economy, Trade and Industry, Contractor CICS Co., Ltd.

### Summary of the Invention

### Problems to be Solved by the Invention

The target for neutron generation having a three-layer structure of substrate/palladium layer/lithium layer does not have any problem in its basic function. However, according to the present inventors, when such a conventional target for neutron generation as described above is subjected to long-term operation, its neutron generation capability may decrease, or lithium layer separation may occur.

The present invention has been accomplished in view of the above circumstances, and provides a target for neutron generation including a lithium layer, which does not degrade in performance regardless of long-term operation, and in which lithium layer separation can be suppressed more than before. Means for Solving the Problems

To solve the problems described above, first, the present inventors have examined factors of performance degradation in conventional targets for neutron generation having a three-layer structure of substrate/palladium layer/lithium layer. Then, as the factor, they have found out the influence of alloy formation at the interface between a lithium layer and a palladium layer.

During proton irradiation for neutron generation, the surface temperature of the target may reach near 100 to 140°C. This target surface temperature is not usually high enough to form an alloy between or among different metals. However, in a lithium-palladium state diagram, there exists a region in which an eutectic alloy is formed at a eutectic temperature of 145°C. Therefore, by the combination of lithium and palladium, eutectic alloy may be formed.

Since a lithium-palladium eutectic alloy, which is a heterophase, inhibits hydrogen entered lithium from reaching the palladium layer, it reduces the function of the palladium layer and decreases the neutron generation capability of the target. Additionally, the heterophase decreases the adhesion strength between the palladium layer and the lithium layer. A palladium layer undergoes dimensional changes, that is, it expands during hydrogen absorption and shrinks during hydrogen release, and such a decrease in adhesion strength causes separation during dimensional changes in the palladium layer.

From this discussion, the present inventors have considered that, to maintain the capability of the target and prevent separation, suppression of the formation of an eutectic alloy at the interface between a lithium layer and a palladium layer is necessary. As a result of further examination, they found that, to avoid the direct contact between a lithium layer and a palladium layer, it is preferable to provide a predetermined barrier layer between the two layers, and arrived at the present invention.

Accordingly, the present invention is a target for neutron generation including a substrate coated with a palladium layer and a lithium layer such that a surface of the lithium layer is arranged to be irradiated with charged particles to generate neutrons, and the target further includes, between the palladium layer and the lithium layer, a barrier layer made of a metal that does not form an eutectic alloy with either palladium or lithium, wherein the barrier layer is made of a metal selected from copper, iron, nickel, cobalt, titanium, and zirconium or an alloy containing any of these metals, and wherein the barrier layer has a thickness of 0.5 to 5 µm.

Hereinafter, each element of the present invention will be described in further detail. Since the target for neutron generation of the present invention has a four-layer structure including a substrate, a palladium layer, a barrier layer, and a lithium layer, each element will be described in detail.

The substrate is preferably made of copper as before. As described above, although the target surface temperature as a result of proton irradiation is relatively low, the target needs to be cooled. Copper has high heat conductivity and thus is a metal suitable as a water-cooled substrate. Its workability is also excellent.

The palladium layer same as before are also applicable. A palladium layer is generally formed on the substrate by a thin-film formation technique such as plating or sputtering. The thickness of the palladium layer is preferably 20 to 100 µm. When the thickness is less than 20 µm, hydrogen cannot be sufficiently absorbed from the lithium layer. When the thickness is more than 100 µm, the water-cooling effect of the copper substrate decreases.

On the palladium layer, a barrier layer, which is a feature of the present invention, is formed. A constituent material for the barrier layer is a metal selected from copper, iron, cobalt, nickel, titanium, and zirconium or an alloy containing any of these metals. The barrier layer contacts both the palladium layer and the lithium layer. Therefore, a material that forms an alloy phase with these metals at the surface temperature at the time of operating the target (near 100 to 140°C) cannot be applied. The metals do not form an alloy phase with either palladium or lithium in this temperature zone. Additionally, iron, titanium, and zirconium have relatively high hydrogen solubility as compared with other metals and thus are advantageous in that the blistering of the barrier layer itself does not occur.

With respect to the thickness of the barrier layer, it is necessary to consider its function and the influence of hydrogen that enters the lithium layer. That is, when the barrier layer is thin, the atomic diffusion between the palladium layer and the lithium layer cannot be suppressed, which allows the formation of an alloy phase. Meanwhile, although the constituent metals for the barrier layer have relatively high hydrogen solubility as compared with other metals, their hydrogen solubility is often lower as compared with palladium. Therefore, when the barrier layer is excessively thick, hydrogen is accumulated in the barrier layer and gasified, to cause blistering. From the above points of view, the thickness of the barrier layer is within a range of 0.5 to 5 µm.

As the lithium layer to serve as a functional surface of the target for neutron generation, those same as before are applicable. The thickness of the lithium layer is preferably 20 to 200 µm.

The shape of the target for neutron generation of the present invention is not limited. A shape optimized for the incidence state of charged particles, for example, plate shape (disc shape), conical shape and cylindrical shape, can be selected.

In a method for producing the target for neutron generation of the present invention, a substrate of a predetermined shape is coated with a palladium layer, a barrier layer, and a lithium layer. As a method for forming each coating layer, plating, sputtering, vapor deposition, thermal spraying, or the like is applied. Particularly, for a palladium layer, plating is preferable, and for a barrier layer, plating is preferable in the case of copper, iron, cobalt, and nickel, while cold-spraying is preferable for titanium and zirconium. Additionally, for a lithium layer, deposition is mainly applied.

The palladium layer, barrier layer, and lithium layer may be formed successively in a continuous manner. Additionally, to freely form a lithium layer according to the handleability of lithium or the device prior to the use of the device, a structure having no lithium layer may be prepared, and then form a lithium layer. The structure includes a substrate, a palladium layer formed on the substrate, and a barrier layer formed on the palladium layer (made of a metal that does not form an eutectic alloy with either of palladium or lithium, such as copper, iron, nickel, cobalt, titanium, or zirconium, as described above). Advantageous Effects of the Invention

As described above, in the target for neutron generation of the present invention, the barrier layer provided between the lithium layer and the palladium layer suppresses the formation of a lithium-palladium eutectic alloy, whereby a decrease in neutron generation efficiency and lithium layer separation caused by a eutectic alloy can be suppressed. The present invention enables the maintenance of the efficiency and durability of a target for neutron generation, and serves as a foundation for its long-term operation.

### Brief Description of the Drawings

Fig. 1 is a diagram illustrating a sectional structure of a conventional target for neutron generation.
Fig. 2 is a cross-sectional photograph of a simulation sample of a comparative example after heating at 140°C.

### Description of Embodiments

Hereinafter, embodiments of the present invention will be described. In this embodiment, first, simulation samples having various kinds of metal layers as barrier layers between lithium and palladium were produced, and the effects of the barrier layers were examined.

For each sample, a metal film of copper, iron, nickel, cobalt, titanium, or zirconium was formed as a barrier layer on a palladium plate (dimension: 20 mm × 20 mm, 2 mm thick) by plating, sputtering, or pressure welding, and a lithium of 5 mm × 5 mm, 2 mm thick, was forge-welded on the film at normal temperature. The sample was then heated at 100°C and 140°C for 1.5 h and 5 h in an Ar atmosphere. After heating, the sample was immersed in water to dissolve and remove lithium, and then the condition of the sample surface was examined. When discoloration was observed on the sample surface at this time, samples in which eutectic alloy formation occurred were rated as "×". The test results are shown in Table 1.

**[Table 1]**

| | Sample structure | Film formation method | Barrier layer thickness (µm) | Eutectic alloy formation | | | |
|---|---|---|---|---|---|---|---|
| | | | | 100°C | | 140°C | |
| | | | | 1.5 h | 5 h | 1.5 h | 5 h |
| Example 1 | Li/Cu/Pd | Plating | 4.8 | ○ | ○ | ○ | ○ |
| Example 2 | Li/Fe/Pd | Pressure weldinq | 4.6 | ○ | ○ | ○ | ○ |
| Example 3 | Li/Ni/Pd | Platinq | 1.0 | ○ | ○ | ○ | ○ |
| Example 4 | Li/Co/Pd | Sputtering | 0.6 | ○ | ○ | ○ | ○ |
| Example 5 | Li/Ti/Pd | Pressure welding | 4.8 | ○ | ○ | ○ | ○ |
| Example 6 | Li/Zr/Pd | Sputtering | 0.6 | ○ | ○ | ○ | ○ |
| Comparative Example | Li/Pd (No barrier layer) | - | - | × | × | × | × |

| | | | | | | | |
|---|---|---|---|---|---|---|---|
| ○: No evidence of eutectic alloy formation was observed. ×: Evidence of eutectic alloy formation (blackening) was observed. | | | | | | | |

As shown in Table 1, in the samples having various metals, such as copper, inserted as barrier layers between lithium and palladium, evidence of eutectic alloy formation was not observed on the surface (barrier layer surface) even after heating at 140°C. Meanwhile, in the samples having no barrier layer (comparative example), discoloration to black was observed on the surface (palladium surface). It appears that this is attributable to the formation of an eutectic alloy of lithium and palladium. This discoloration was also observed at 100°C, but the color was lighter than when heated at 140°C. It appears that although the heating temperature of 100°C is lower than the eutectic temperature, an eutectic alloy was slightly formed.

Fig. 2 shows a cross-sectional photograph of the comparative example after heating at 140°C. A phase that is believed to be an eutectic alloy is formed between the lithium layer and the palladium layer. From the above, it was confirmed that when the contact between lithium and palladium is broken by a barrier layer, the formation of an eutectic alloy can be suppressed.

### Second Embodiment:

Here, a target for neutron generation having a shape and dimension for actual operation was produced to confirm the effectiveness of barrier layer formation. A disk-shaped copper substrate of φ 135 mm × 8 mm thickness was prepared. A palladium layer was formed over the entire surface by plating, and a copper layer was formed as a barrier layer in a central φ 73 mm region of the substrate by plating. Further, a lithium layer was formed in a central φ 60 mm region of the substrate by vapor deposition. The thickness of each layer was as follows: palladium layer: 20 µm, barrier layer (copper): 3 µm, lithium layer: 100 µm.

This target for neutron generation was irradiated with a proton beam as charged particles to generate neutrons. An electrostatic accelerator was used as a proton beam accelerator, and the target was irradiated with a proton beam having an energy value of 2.5 MeV for 3 hours at a current density of 63%. The dose of neutrons generated during the irradiation was measured by a proportional counter, and no attenuation of the neutron dose was observed. Then, after the completion of irradiation, the condition of the target surface was observed, and there was no significant consumption or separation of lithium. Accordingly, it was confirmed that the target for neutron generation of the present invention has durability and stability against continuous irradiation with a proton beam.

### Industrial Applicability

The target for neutron generation of the present invention includes a barrier layer added between a lithium layer and a palladium layer, to suppress the formation of a lithium-palladium eutectic alloy. In the present invention, palladium sufficiently exerts its original function to maintain the neutron generation efficiency, and lithium layer separation can be suppressed. The target for neutron generation of the present invention promotes the implementation of neutron capture therapy, such as boron neutron capture therapy (BNCT), outside a nuclear reactor facility, and thus is useful to achieve practical application of the neutron capture therapy.

## Claims

1. A target for neutron generation, comprising a substrate coated with a palladium layer and a lithium layer such that a surface of the lithium layer is arranged to be irradiated with charged particles to generate neutrons, the target for neutron generation **characterised in that** it further comprises, between the palladium layer and the lithium layer, a barrier layer made of a metal that does not form an eutectic alloy with either palladium or lithium, wherein the barrier layer is made of a metal selected from copper, iron, nickel, cobalt, titanium, and zirconium or an alloy containing any of these metals, and wherein the barrier layer has a thickness of 0.5 to 5 µm.

## Patentansprüche

1. Target zur Neutronenerzeugung, umfassend ein Substrat, das mit einer Palladiumschicht und einer Lithiumschicht beschichtet ist, sodass eine Oberfläche der Lithiumschicht so angeordnet ist, dass sie mit geladenen Teilchen bestrahlt werden kann, um Neutronen zu erzeugen,
wobei das Target für Neutronenerzeugung **dadurch gekennzeichnet ist, dass** es weiterhin zwischen der Palladiumschicht und der Lithiumschicht eine Barriereschicht umfasst, hergestellt aus einem Metall, das weder mit Palladium noch mit Lithium eine eutektische Legierung bildet, worin die Barriereschicht hergestellt ist aus einem Metall, ausgewählt aus Kupfer, Eisen, Nickel, Cobalt, Titan und Zirkonium oder einer Legierung, die eines dieser Metalle enthält, und worin die Barriereschicht eine Dicke von 0,5 bis 5 µm hat.

## Revendications

1. Cible pour génération de neutrons, comprenant un substrat enrobé d'une couche de palladium et d'une couche de lithium de telle sorte qu'une surface de la couche de lithium est agencée pour être irradiée de particules chargées pour générer des neutrons, la cible pour génération de neutrons étant **caractérisée en ce qu'**elle comprend en outre, entre la couche de palladium et la couche de lithium, une couche barrière composée d'un métal qui ne forme pas un alliage eutectique avec soit le palladium, soit le lithium, dans laquelle la couche barrière est composée d'un métal sélectionné parmi le cuivre, le fer, le nickel, le cobalt, le titane et le zirconium ou un alliage contenant l'un quelconque de ces métaux, et dans laquelle la couche barrière présente une épaisseur de 0,5 à 5 µm.
